(19) Europäisches Patentamt
European Patent Office
Office européen des brevets

(11) Veröffentlichungsnummer : **0 141 960**
**B1**

(12) # EUROPÄISCHE PATENTSCHRIFT

(45) Veröffentlichungstag der Patentschrift : **06.05.87**

(51) Int. Cl.[4] : **C 07 D 335/02**

(21) Anmeldenummer : **84110797.2**

(22) Anmeldetag : **11.09.84**

(54) **Verfahren zur Herstellung von 3-Formyl-tetrahydrothiopyranen.**

(30) Priorität : **17.09.83 DE 3333678**

(43) Veröffentlichungstag der Anmeldung : **22.05.85 Patentblatt 85/21**

(45) Bekanntmachung des Hinweises auf die Patenterteilung : **06.05.87 Patentblatt 87/19**

(84) Benannte Vertragsstaaten : **AT BE CH DE FR GB IT LI NL**

(56) Entgegenhaltungen :
**DE-A- 1 919 504**
**GB-A- 1 479 916**
**CHEMICAL ABSTRACTS, Vol. 87, no. 3, 18. Juli 1977, Columbus, Ohio, USA MC INTOSH, JOHN M.; KHALIL, HAMDY "Phase-transfer catalyzed syntheses. 5-Thiacyclohexene-carboxaldehydes and 3,4-epoxy-2,5-dihydrothiophenes." Seite 636, Spalte 1, Zusammenfassung-No. 23096w**

(73) Patentinhaber : **BASF Aktiengesellschaft**
**Carl-Bosch-Strasse 38**
**D-6700 Ludwigshafen (DE)**

(72) Erfinder : **Goetz, Norbert, Dr.**
**Schoefferstrasse 25**
**D-6520 Worms 1 (DE)**
Erfinder : **Jahn, Dieter, Dr.**
**Burgunder Weg 8**
**D-6803 Edingen-Neckarhausen (DE)**
Erfinder : **Reissenweber, Gernot, Dr.**
**Drosselstrasse 15**
**D-6737 Boehl-Iggelheim (DE)**
Erfinder : **Eckhardt, Heinz, Dr.**
**Ruedigerstrasse 7**
**D-6700 Ludwigshafen (DE)**

**EP 0 141 960 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von 3-Formyl-tetrahydrothiopyranen durch Hydrierung von 3-Formyl-5,6-dihydro-2H-thiopyranen in Gegenwart von Katalysatoren, die Nickel, Kobalt, Platin, Kupfer und/oder Silber enthalten, wobei gegebenenfalls die 3-Formyl-5,6-dihydro-2H-thiopyrane durch Umsetzung von Bis-(β-formylethyl)-sulfiden und/oder 3-Formyl-4-hydroxy-tetrahydrothiopyranen in Gegenwart von sauren Katalysatoren und gegebenenfalls diese Bis-(β-formylethyl)-sulfide und/oder 3-Formyl-4-hydroxytetrahydrothiopyrane durch Umsetzung von Acroleinen mit Schwefelwasserstoff in Gegenwart von a) basischen Katalysatoren und von Methylenchlorid, aromatischen Kohlenwasserstoffen und/oder 1,1,2-Trichlorethan als Lösungsmitteln und/oder b) in Gegenwart von Carbonsäureamiden hergestellt werden.

Es ist bekannt, daß 3-Formyl-5,6-dihydro-2H-thiopyran aus Acrolein und Schwefelwasserstoff in Gegenwart von Kupferspänen und Triethylamin bei —10 °C (Z. Lebensm. Unters. Forsch. *170*, S. 34 bis 35 (1980)) hergestellt werden kann. Ebenfalls zeigt die DE-AS 19 19 504 eine Herstellung in 2 Stufen, nämlich eine Reaktion in Gegenwart von Basen und Lösungsmitteln bei —10 bis + 150 °C und dann die Umsetzung des Reaktionsprodukts mit einer starken Säure bei 60 bis 160 °C. Die Beispiele zeigen eine Arbeitsweise unter Druck mit Chloroform und Methanol als Lösungsmitteln, tertiären Aminen als Basen und Phosphorsäure bzw. Schwefelsäure als Säuren.

Die Herstellung des gesättigten Aldehyds, 3-Formyl-tetrahydrothiopyran, wurde bisher nur in der britischen Patentschrift 1 479 916 beschrieben. Zunächst wurde aus 3-Chlorpropionaldehyd-diethylacetal und Natriumhydrogensulfid 3-Formyl-5,6-dihydro-2H-thiopyran hergestellt, das dann an Palladiumkatalysatoren auf Kohleträgern zum gesättigten Aldehyd hydriert wurde. Die Beispiele zeigen, daß hohe Katalysatormengen benötigt werden ; auf 3,2 g 3-Formyl-5,6-dihydro-2H-thiopyran wurden 1,5 g Katalysator mit 10 % Palladium auf Kohle eingesetzt.

Die Überführung von ungesättigten Sulfiden in gesättigte Sulfide durch Hydrierung an Palladium/Holzkohle-Katalysatoren ist bekannt. Es wird darauf hingewiesen, daß eine Hydrierung an Schwermetallkatalysatoren unter den üblichen Bedingungen, nämlich bei hohen Temperaturen unter Druck, zu erheblicher Hydrogenolyse führt (J. Chem. Soc., S. 2888 bis 2890 (1958)).

Weiterhin ist beschrieben, daß zur partiellen Hydrierung von α,β-ungesättigten Aldehyden zu den gesättigten Aldehyden vorzugsweise Palladiumkatalysatoren Verwendung finden (Rylander, Catalytic Hydrogenation over Platinum Metals (Academic Press, New York, 1967), S. 107 bis 108). Die DE-OS 28 32 699 beschreibt Katalysatoren, die aus Palladium und Verbindungen eines seltenen Erdmetalls bestehen, und weist darauf hin, daß bei dieser Kombination trotz der empfindlichen Aldehydgruppe eine Perhydrierung olefinisch ungesättigter Aldehyde zu den entsprechenden gesättigten Alkoholen vermieden wird. Die Literatur weist darauf hin, daß zwar auch Platinkatalysatoren im Falle aliphatischer, ungesättigter Aldehyde in Frage kommen, in allen Fällen aber Palladium der Katalysator der Wahl ist, da nur solche Katalysatoren selektiv die Olefingruppe reduzieren, ohne die Carbonylgruppe wesentlich zu reduzieren. Als Beispiel dafür wird die Reduktion von Citronellal gezeigt, bei dem Palladiumkatalysatoren an der olefinischen Doppelbindung, Nickelkatalysatoren hingegen an der Carbonylgruppe angreifen.

Nickel-Katalysatoren werden durch Schwefelverbindungen, beispielsweise Thiophen, schnell vergiftet (H. D. Hartough, Thiophene and Its Derivatives (Interscience Publishers, New York, 1952, S. 167 bis 168). Es wird darauf hingewiesen, daß Nickel- und Kobaltkatalysatoren zur Entschwefelung von organischen Verbindungen dienen, wobei Schwefelwasserstoff entsteht. Auch Platinkatalysatoren werden durch Thiophen vergiftet. Sowohl Thiophen- wie Thiolanverbindungen können bei der Hydrierung durch Kupfer-, Platin- und Nickelkatalysatoren gespalten werden. Auch ein Artikel in Angew. Makromol. Chem. *52* (1976), S. 63 bis 70, zeigt Nickelkatalysatoren als wirksame Entschwefelungskatalysatoren. Freifelder, Practical Catalytic Hydrogenation (Wiley Interscience, New York, 1971), S. 153 bis 154 empfiehlt ebenfalls Palladiumkatalysatoren für die Hydrierung von ungesättigten Aldehyden und zeigt, daß Platin- und Nickelkatalysatoren ungeeignet sind, bzw. Nickelkatalysatoren die Perhydrierung unterstützen.

Die DE-AS 19 19 504 beschreibt die Herstellung von 3-Formyl-tetrahydrothiopyran (1-Thiacyclo-$\Delta^3$-hexenal (3)) durch Umsetzung von Acrolein und Schwefelwasserstoff in Gegenwart von organischen Basen, gegebenenfalls in Gegenwart von organischen Lösungsmitteln, und anschließender Nachbehandlung des Umsetzungsproduktes mit einer starken Säure. In den Beispielen werden Drücke von bis zu 6,5 atü, Methanol oder Chloroform als Lösungsmittel und tertiäre Amine als Basen beschrieben ; der Schwefelwasserstoff und die Base werden stets vorgelegt und Acrolein dann dem Gemisch zugegeben.

Es wurde nun gefunden, daß man 3-Formyltetrahydrothiopyrane der Formel

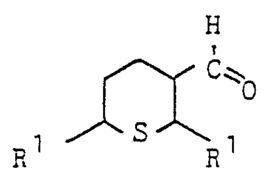

(I)

worin die einzelnen Reste $R^1$ jeweils ein Wasserstoffatom oder einen Alkylrest mit 1 bis 8 Kohlenstoffatomen, der durch unter den Reaktionsbedingungen inerte Gruppen substituiert sein kann, bedeuten, durch Hydrierung von 3-Formyl-5,6-dihydro-2H-thiopyranen in Gegenwart von Katalysatoren, dadurch gekennzeichnet, daß man 3-Formyl-5,6-dihydro-2H-thiopyrane der Formel

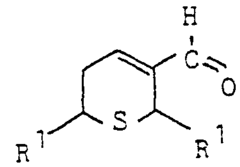

(II)

worin $R^1$ die vorgenannte Bedeutung besitzt, in Gegenwart von Katalysatoren, die Nickel, Kobalt, Platin, Kupfer und/oder Silber enthalten, hydriert.

Es wurde weiterhin gefunden, daß man das Verfahren vorteilhaft durchführt, wenn man in einer ersten Stufe Bis-(β-formylethyl)-sulfide der Formel

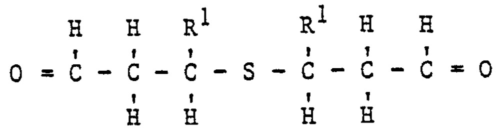

(III)

worin $R^1$ die vorgenannte Bedeutung besitzt, und/oder 3-Formyl-4-hydroxytetrahydrothiopyrane der Formel

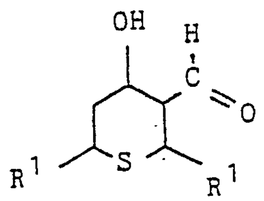

(IV)

worin $R^1$ die vorgenannte Bedeutung besitzt, in Gegenwart saurer Katalysatoren umsetzt und dann in einer zweiten Stufe die so gebildeten 3-Formyl-5,6-dihydro-2H-thiopyrane der Formel

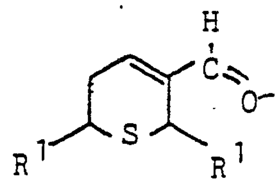

(II)

worin $R^1$ die vorgenannte Bedeutung besitzt, in Gegenwart von Katalysatoren, die Nickel, Kobalt, Platin, Kupfer und/oder Silber enthalten, hydriert.

Es wurde weiterhin gefunden, daß man das Verfahren vorteilhaft ausführt, wenn man in einer ersten Stufe Acroleine der Formel

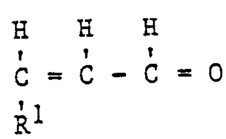

(V)

worin $R^1$ die vorgenannte Bedeutung besitzt, mit Schwefelwasserstoff in Gegenwart von

a) basischen Katalysatoren und von Methylenchlorid, aromatischen Kohlenwasserstoffen und/oder 1,1,2-Trichlorethan als Lösungsmittel und/oder

b) Carbonsäureamiden

umsetzt und dann in einer zweiten Stufe das so erhaltene Gemisch von Bis-(β-formylethyl)-sulfiden der Formel

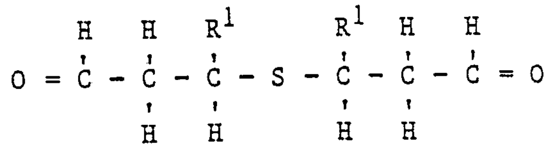

(III)

worin $R^1$ die vorgenannte Bedeutung besitzt, und 3-Formyl-4-hydroxytetrahydrothiopyranen der Formel

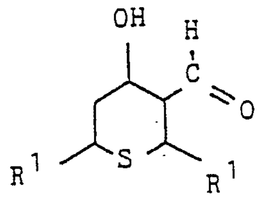

(IV)

worin $R^1$ die vorgenannte Bedeutung besitzt, in Gegenwart saurer Katalysatoren umsetzt und dann in einer dritten Stufe die so gebildeten 3-Formyl-5,6-dihydro-2H-thiopyrane der Formel

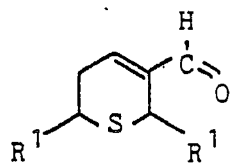

(II)

worin $R^1$ die vorgenannte Bedeutung besitzt, in Gegenwart von Katalysatoren, die Nickel, Kobalt, Platin, Kupfer und/oder Silber enthalten, hydriert.

Weiterhin wurde gefunden, daß man das Verfahren vorteilhaft ausführt, wenn als Hydrierkatalysatoren Nickel-, Kobalt-, Platin-, Kupfer- und/oder Silber-Katalysatoren, die noch dazu basische Oxide enthalten, verwendet werden.

Die Umsetzungen können im Fall der Verwendung von Acrolein und der dreistufigen erfindungsgemäßen Verfahrensweise durch folgende Formeln beschrieben werden :

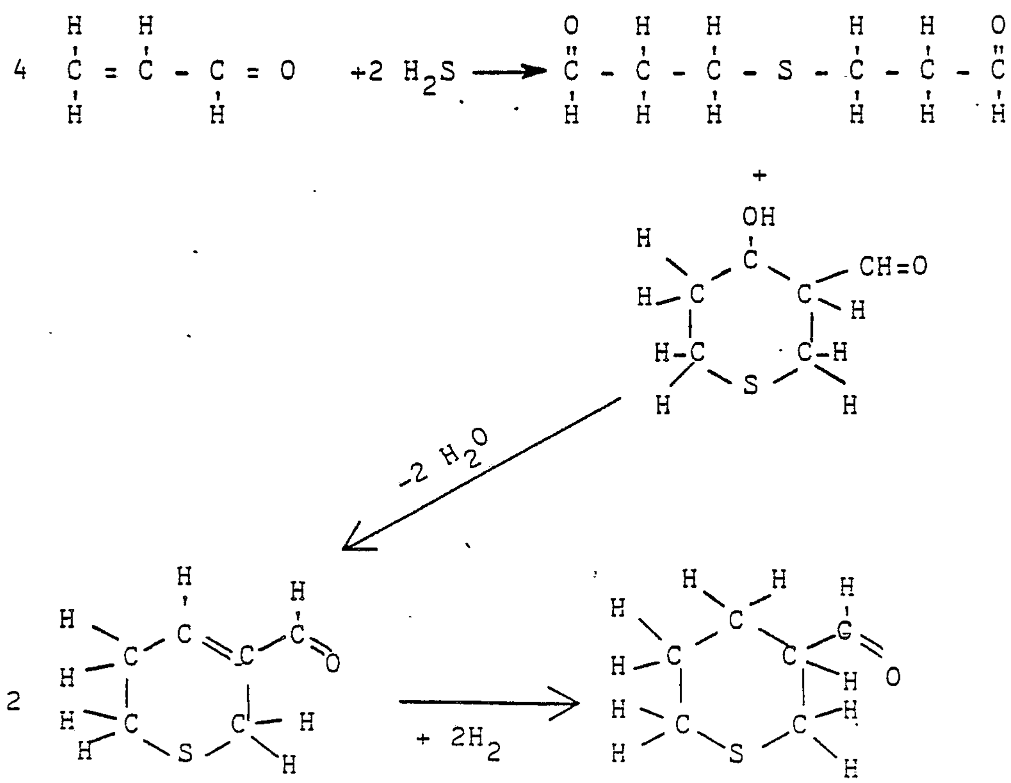


Im Hinblick auf die bekannten Verfahren liefert das Verfahren nach der Erfindung auf einfacherem und wirtschaftlicherem Wege 3-Formyl-tetrahydrothiopyrane in besserer Ausbeute und Reinheit. Alle diese vorteilhaften Ergebnisse sind im Hinblick auf den Stand der Technik überraschend. So konnte nicht erwartet werden, daß bei der Hydrierung von 3-Formyl-5,6-dihydro-2H-thiopyranen Palladiumkatalysatoren schnell vergiftet werden (Vergleichsbeispiel 6), während die erfindungsgemäßen Katalysatoren, insbesondere Nickelkatalysatoren, sehr selektiv hydrieren und ohne nennenswerten Aktivitätsverlust wiederholt eingesetzt werden können. Im Hinblick auf die Lehre von Hartough (loc. cit.) hätte man eine rasche Vergiftung der erfindungsgemäßen Nickelkatalysatoren vermutet. Insbesondere war es mit Bezug auf die DE-OS 28 32 699 und die Veröffentlichung von Freifelder (loc. cit.) überraschend, daß keine erhebliche Perhydrierung der Thiopyrane zu den entsprechenden Alkoholen stattfindet. Auf jeden Fall hätte man im Hinblick auf diese Veröffentlichung, das Werk von Hartough (loc. cit.) und den Artikel in der Angewandten Makromolekularen Chemie (loc. cit.) mit einem erheblichen Aktivitätsabfall des Katalysators, der Spaltung des Thiopyranrings, der Bildung von Schwefelwasserstoff und von heterogenen Gemischen zahlreicher Spalt- und Nebenprodukte gerechnet.

Die Umsetzung von Acroleinen mit Schwefelwasserstoff zu einem Gemisch von Bis-(β-formylethyl)-sulfiden III und 3-Formyl-4-hydroxy-tetrahydrothiopyranen IV in Gegenwart von tertiären Aminen kann, im Hinblick auf die DE-AS 19 19 504 überraschend, auch mit Vorlage des Acroleins und Zuführung des Schwefelwasserstoffs oder zweckmäßiger unter gleichzeitiger Zufuhr von Acrolein und Schwefelwasserstoff in den Reaktionsraum drucklos bei Temperaturen zwischen 10 und 60 °C erfolgen. Ebenfalls konnte nicht mit Bezug auf diese Veröffentlichung vermutet werden, daß nach dem erfindungsgemäßen Verfahren Methylenchlorid, aromatische Kohlenwasserstoffe und bevorzugt 1,1,2-Trichlorethan als Lösungsmittel Verwendung finden, während Alkohole schlechtere Ergebnisse liefern und mit 1,1,1-Trichlorethan im Vergleich zum isomeren 1,1,2-Trichlorethan überraschend kein wesentlicher Anteil an Ausgangsstoffen III und IV sondern nur unerwünschte polymere Produkte erhalten werden (Vergleichsversuch 3).

Die Umsetzung (Hydrierung) kann mit Wasserstoff in stöchiometrischer Menge oder zweckmäßig im Überschuß, vorteilhaft in einer Menge von 1 bis 50, insbesondere 1 bis 10 Mol Wasserstoff je Mol Ausgangsstoff II durchgeführt werden. Bevorzugte Ausgangsstoffe II und Stoffe III, IV, V und dementsprechend bevorzugte Endstoffe I sind solche, in deren Formeln die einzelnen Reste $R^1$ jeweils ein Wasserstoffatom oder einen Alkylrest mit 1 bis 4 Kohlenstoffatomen bedeuten. Die vorgenannten Reste können noch durch unter den Reaktionsbedingungen inerte Gruppen, z. B. Alkylgruppen mit 1 bis 4 Kohlenstoffatomen, substituiert sein.

So konnten beispielsweise folgende Thiopyrane als Ausgangsstoffe II verwendet werden : 2-Methyl, 2-Ethyl, 2-Propyl, 2-Isopropyl, 2-Butyl, 2-Isobutyl, 2-sek.-Butyl, 2-tert.-Butyl-3-formyl-5,6-Dihydro-2H-thiopyran ; durch vorgenannte Gruppen statt in 2-Stellung in 6-Stellung substituierte 3-Formyl-5,6-dihydro-2H-thiopyrane ; durch vorgenannte Gruppen in 2- und 6-Stellung disubstituierte 3-Formyl-5,6-dihydro-2H-thiopyrane ; bevorzugt 3-Formyl-5,6-dihydro-2H-thiopyran.

Die Umsetzung (Hydrierung) wird in der Regel bei einer Temperatur von 10 bis 180 °C, vorzugsweise 50 bis 150 °C, drucklos, unter Unterdruck oder bevorzugt unter Überdruck, zweckmäßig bei einem Druck zwischen 1 und 300 bar, vorteilhaft zwischen 1 und 205 bar, bevorzugt zwischen 1 und 155 bar, vorzugsweise zwischen 1 und 100 bar, insbesondere von 5 bis 50 bar, kontinuierlich oder diskontinuierlich durchgeführt. Zweckmäßig verwendet man unter den Reaktionsbedingungen inerte, organische Lösungsmittel. Als Lösungsmittel kommen z. B. in Frage Ether, z. B. Ethylpropylether, Methyl-tert.-butylether, n-Butylethylether, Di-n-butylether, Diisobutylether, Diisopropylether, Diethylether, Diethylenglykoldimethylether, Tetrahydrofuran, Dioxan ; Alkanole und Cycloalkanole wie Ethanol, Methanol, n-Butanol, Isobutanol, tert.-Butanol, Glykol, 3-Methoxypropanol, sek.-Butanol, n-Propanol, Isopropanol, Cyclohexanol, Ethylenglykolmonoethylether, 2-Ethylhexanol, Methylglykol, n-Hexanol, Isohexylalkohol, Isoheptylalkohol, n-Heptanol, Ethylbutanol, Nonylalkohol, Dodecylalkohol, Methylcyclohexanol, insbesondere solche mit 1 bis 4 Kohlenstoffatomen ; tert. Amine, wie N-Methylpiperidin, N-Methylmorpholin ; Carbonsäureamide, wie N,N-Dimethylbenzamid, N,N-Dimethylformamid, N,N-Dimethylacetamid, N,N-Dimethylphenylacetamid, N,N-Dimethylcyclohexancarbonsäureamid, N,N-Dimethylpropionsäureamid und homologue N-Methylcarbonsäurepiperidide, N-Methyl-carbonsäurepyrrolidide ; entsprechende N,N-Diethyl-, N,N-Dipropyl-, N,N-diisopropyl-, N,N-Diisobutyl-, N,N-Dibenzyl, N,N-Diphenyl-, N-Methyl-N-phenyl-, N-Cyclohexyl-N-methyl-, N-Ethyl-N-tert.-butyl-verbindungen ; N-Methyl-formanilid-, N-Ethyl-piperidon-(6), N-Methylpyrrolidon, Tetramethylharnstoff ; bevorzugt Ester wie Methylacetat, Ethylacetat, n-Propylacetat, Isobutylformiat, Methylpropionat, n-Butylacetat, Ethylformiat, Phthalsäuremethylester, Benzoesäuremethylester, Phenylacetat ; entsprechende Gemische. Zweckmäßig verwendet man das Lösungsmittel in einer Menge von 10 bis 10 000 Gew.-%, vorzugsweise von 10 bis 1 000 Gew.-%, bezogen auf Ausgangstoff II.

Zur Herstellung der Katalysatoren für die Hydrierung gemäß dem Verfahren der Erfindung finden folgende Metalle Verwendung : Nickel, Kobalt, Platin, Kupfer, Silber. Die Katalysatoren können trägerlos, wie beispielsweise Raney-Nickel, Raney-Kobalt, oder als Trägerkatalysatoren eingesetzt werden. Die trägerlosen Katalysatoren können auch Verbindungen der erfindungsgemäßen Metalle, bevorzugt ihre Oxide sein. Als Träger eignen sich beispielsweise Kohle, Kieselgel, Aluminiumsilikat oder Aluminiumoxid. Die Herstellung solcher Trägerkatalysatoren kann in beliebiger Weise, z. B. durch Tränken des Trägers mit entsprechenden Lösungen der Metallsalze, durch Verkneten bzw. Mischen unter Vermahlen der Komponenten, erfolgen. Bezüglich Details der Herstellung von Katalysatoren, insbesondere Trägerkatalysatoren, wird auf Houben-Weyl, Methoden der Organischen Chemie, Band 4/2, Seiten 137 ff. verwiesen. Bei Trägerkatalysatoren beträgt der Metallgehalt des Katalysators, bezogen auf die Gewichtsmenge

Trägermaterial, gewöhnlich von 0,05 bis 19,5, vorzugsweise 0,5 bis 15 Gew.-%. Das erfindungsgemäße Metall (Ni, Co, Cu, Pt, Ag) des Hydrierkatalysators wird in der Regel in Mengen von 0,1 bis 100, insbesondere 0,5 bis 20 Gew.%, bezogen auf Ausgangsstoff II, verwendet.

In einer bevorzugten Ausführungsform werden in den Katalysatoren neben den erfindungsgemäßen Metallen bzw. Metallverbindungen noch basische Oxide, in der Regel basische Oxide von Metallen der Gruppen IIa und IIIa des periodischen Systems, vorteilhaft Oxide das des Calciums, Magnesiums und/oder der seltenen Erden, insbesondere des Praeseodyms, Cers, verwendet. Diese Gruppen beziehen sich in ihrer Anordnung auf D'Ans-Lax, Taschenbuch für Chemiker und Physiker (Springer, Berlin, 1967), Band I, Seite 63). Der Gehalt an basischen Metalloxiden im Katalysator beträgt zweckmäßig zwischen 0,5 und 90 Gew.%, vorzugsweise von 0,5 bis 30 Gew.%, bezogen auf den Katalysator bzw. auf den Trägerkatalysator, wobei das basische Metalloxid, z. B. Magnesiumoxid, selbst den Träger oder einen Anteil des Trägers darstellen kann.

Man verwendet den Katalysator bzw. Trägerkatalysator (kontinuierliche oder dikontinuierliche Arbeitsweise), zweckmäßig in Form von Strängen oder als Pulver.

Die Umsetzung kann wie folgt durchgeführt werden : Ein Gemisch von Ausgangsstoff II, Wasserstoff, Katalysator und gegebenenfalls Lösungsmittel wird bei der Reaktionstemperatur und dem Reaktionsdruck umgesetzt. Man kann den Wasserstoff kontinuierlich oder diskontinuierlich der Reaktion zuführen und/oder den Katalysator selbst nach einer bestimmten Reaktionszeit wieder frisch mit Wasserstoff beladen. In der Regel werden dem Reaktionsgemisch am Anfang und im Verlauf der Umsetzung solche Mengen an Wasserstoff zugeführt, daß sich bei der Umsetzungstemperatur stets ein entsprechender Reaktionsdruck einstellt. Zur entsprechenden Druckeinstellung können auch inerte Gasewie Stickstoff neben Wasserstoff verwendet werden. Beispielsweise gibt man den Ausgangsstoff II mit dem Lösungsmittel in den Reaktor, setzt den Hydrierkatalysator zu und spült den Reaktionsraum mit Stickstoff. Dann wird Wasserstoff bis zu vorgenanntem Reaktionsdruck eingepreßt. Nun wird das Reaktionsgemisch auf vorgenannte Temperatur gebracht und solange bei dieser Temperatur unter Einleitung von weiterem Wasserstoff gehalten, bis kein Wasserstoff mehr durch die Reaktion verbraucht wird. Nun wird das Reaktionsgemisch abgekühlt und filtriert. Aus dem Filtrat wird der Endstoff nach den üblichen Methoden, z. B. durch Destillation, abgetrennt.

Der Ausgangsstoff II kann in beliebiger Weise, z. B. nach den in der DE-AS 19 19 504 beschriebenen Verfahren, hergestellt werden. In zwei bevorzugten Ausführungsformen wird ein Acrolein V mit Schwefelwasserstoff in der Ausführungsform a in Gegenwart von basischen Katalysatoren zusammen mit dem Lösungsmittel Methylenchlorid, aromatischen Kohlenwasserstoffen und/oder bevorzugt 1,1,2-Trichlorethan oder in der Ausführungsform b in Gegenwart von gleichzeitig als basische Katalysatoren und Lösungsmittel wirkenden Carbonsäureamiden umgesetzt. Sowohl die Verfahrensweise a wie die Verfahrensweise b wird zweckmäßig bei einer Temperatur von — 15 bis + 60 °C, vorzugsweise von — 8 bis + 40 °C, drucklos, unter Überdruck oder unter Unterdruck, kontinuierlich oder diskontinuierlich durchgeführt. Die für Verfahrensweise a oder b angegebenen Lösungsmitteln verwendet man einzeln oder in entsprechenden Gemisch. Zweckmäßig verwendet man das Lösungsmittel bzw. das Lösungsmittelgemisch in einer Menge von 10 bis 10 000 Gew.%, vorzugsweise von 10 bis 1 000 Gew.%, bezogen auf Ausgangsstoff V. Man kann Ausgangsstoff V und Schwefelwasserstoff in stöchiometrischer Menge oder im Überschuß jeder Komponente von anderen, zweckmäßig in einem Verhältnis von 0,3 bis 10, vorzugsweise 0,5 bis 2 Mol Schwefelwasserstoff je Mol Acrolein V, umsetzen.

Die Umsetzung der Verfahrensweise a) wird in Gegenwart einer basischen Verbindung als Katalysator, vorteilhaft in einer Menge von 0,001 bis 0,1, vorzugsweise von 0,005 bis 0,05 Äquivalenten basischer Verbindung, bezogen auf ein Mol Ausgangsstoff V, durchgeführt. Bevorzugte basische Verbindungen sind tertiäre Amine. Es können aber auch Erdalkaliverbindungen, Ammoniumverbindungen, Alkaliverbindungen, primäre oder sekundäre Amine verwendet werden. Es kommen z. B. als basische Verbindungen in Frage : Kaliumhydroxid, Natriumhydroxid, Kaliumcarbonat, Natriumcarbonat, Natriumbicarbonat, Kaliumbicarbonat, Calciumhydroxid, Calciumoxid, Bariumoxid, Magnesiumhydroxid, Magnesiumoxid, Bariumhydroxid, Calciumcarbonat, Natriumformiat, Natriumacetat, Kaliumformiat, Kaliumacetat, Natriummethylat, Natriumethylat, Kaliummethylat, Kaliumethylat, Trimethylamin, Triethylamin, Tripropylamin, Triisopropylamin, Tributylamin, Triisobutylamin, Tri-sek.-butylamin, Tri-tert.-butylamin, Tribenzylamin, Tricyclohexylamin, Triamylamin, Trihexylamin, N,N-Dimethylanilin, N,N-Diethylanilin, Dimethylamin, Diethylamin, Dipropylamin, Diisopropylamin, Dibutylamin, Diisobutylamin, Pyrrolidon, Piperidin, Pyrrolidin, Imidazol, Pyrrol, N,N'-Dimethylethylendiamin, N,N'-Diethylethylendiamin, Morpholin, Hexamethylenimin, Methylamin, Ethylamin, Propylamin, Isopropylamin, Butylamin, sek.-Butylamin, tert.-Butylamin, Anilin, N-Methylpyrrolidon, N-Ethylpyrrolidon, N-Methylpiperidin, N-Ethylpiperidin, N-Methylpyrrolidin, N-Ethylpyrrolidin, N-Ethylpyrrol, N-Methylmorpholin, N-Ethylmorpholin, N-Methylhexamethylenimin, N-Ethylhexamethylenimin, Pyridin, Chinolin, Pyrimidin.

Bei der Verfahrensweise b) werden die Carbonsäureamide einzeln oder im Gemisch zweckmäßig ohne zusätzliche Lösungsmittel eingesetzt. Gegebenenfalls kann man sie auch zusammen mit aromatischen Kohlenwasserstoffen, 1,1,2-Trichlorethan und/oder Methylenchlorid, zweckmäßig in dem schon für die Verfahrensweise a) genannten Mengenverhältnis, bezogen auf Acrolein V, oder auch zusammen mit den basischen Katalysatoren und einem oder den beiden Lösungsmitteln von a), zweckmäßig unter den vorgenannten Reaktionsbedingungen on a), verwenden. Als Carbonsäureamide kommen aromatische,

araliphatische, cycloaliphatische oder insbesondere aliphatische oder cyclische Carbonsäureamide, am Stickstoffatom unsubstituierte, monosubstituierte oder vorzugsweise disubstituierte Carbonsäureamide in Frage. Bevorzugt sind Amide der Formel

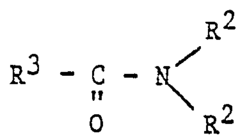

(VI)

worin die einzelnen Reste $R^2$ und $R^3$ gleich oder verschieden sind und jeweils ein Wasserstoffatom, einen Alkylrest mit 1 bis 4 Kohlenstoffatomen, einen Cyclohexylrest, einen Aralkylrest mit 7 bis 12 Kohlenstoffatomen, einen Phenylrest bedeuten, darüber hinaus die Reste $R^2$ und $R^3$ auch zusammen mit dem benachbarten Kohlenstoffatom und Stickstoffatom für Glieder eines 5- oder 6-gliedrigen heterocyclischen Ringes oder die Reste $R^2$ und das beiden benachbarte Stickstoffatom für Glieder eines 5- oder 6-gliedrigen heterocyclischen Ringes stehen können, oder $R^3$ auch den Rest

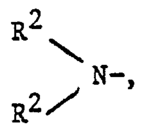

worin die Reste $R^2$ die vorgenannte Bedeutung haben, bezeichnen kann. Die Reste und Ringe können noch durch unter den Reaktionsbedingungen inerte Gruppen, z. B. Alkylgruppen mit 1 bis 3 Kohlenstoffatomen, substituiert sein.

Geeignete Amide sind beispielsweise N,N-Dimethylbenzamid, N,N-Dimethylbuttersäureamid, N,N-Dimethylphenylacetamid, N,N-Dimethylcyclohexancarbonsäureamid, N,N-Dimethylpropionsäureamid und homologes Carbonsäurepiperidid, Carbonsäurepyrrolidid ; entsprechende N,N-Diethyl-, N,N-Dipropyl-, N,N-Diisopropyl-, N,N-Diisobutyl-, N,N-Dibenzyl, N,N-Diphenyl-, N-Methyl-N-Phenyl-, N-Cyclohexyl-N-methyl-, N-Ethyl-N-tert.-butyl-Verbindungen ; M-Methyl-formanilid, N-Ethyl-piperidon-(6), Tetramethylharnstoff ; entsprechende Gemische. Bevorzugt sind Dimethylformamid, Diethylformamid, Dimethylacetamid, Diethylacetamid, N-Methylpyrrolidon.

Bei der Umsetzung nach a) oder b) erhält man Gemische der Ausgangsstoffe III und IV, in einem Verhältnis von 0,1 bis 1 Mol Ausgangsstoff III (Bis-(β-formylethyl)-sulfid) je Mol Ausgangsstoff IV (3-Formyl-4-hydroxytetrahydrothiopyran), die man in üblicher Weise, z. B. durch fraktionierte Destillation, abtrennt und einzeln oder im Gemisch miteinander einer 2. Stufe, der Säurebehandlung, zuführen kann. Zweckmäßiger wird man aber das Reaktionsgemisch der Umsetzung a) oder b) nicht aufarbeiten sondern ihm die Säure zugeben und die Umsetzung der 2. Stufe durchführen. In der Regel verwendet man starke Säuren. Unter starken Säuren werden hier organische oder anorganische, unter den Reaktionsbedingungen inerte Säuren mit einem Säureexponent (pKs) von — 7 bis + 2,16 verstanden ; bezüglich der Definition der Säureexponenten bzw. des pKs-Wertes wird auf Ullmanns Encyklopädie der technischen Chemie, Band 15, Seite 2, verwiesen. Geeignet sind beispielsweise Schwefelsäure, Phosphorsäure, Chlorwasserstoffgas, Ameisensäure, Borsäure, Sulfonsäuren wie Benzol- und p-Toluolsulfonsäure, Trichloressigsäure, Ionenaustauscher wie die in Houben-Weyl, Methoden der Organischen Chemie, Band I/1, Seiten 528 ff. beschriebenen sauren Ionenaustauscher, bevorzugt Polystyrolsulfonsäureharze, Phenolsulfonsäureharze, Polyfluorethylensulfonsäureharze ; oder entsprechende Gemische. Bevorzugte Säuren sind Schwefelsäure, Toluolsulfonsäure oder Phosphorsäure. Die Säure verwendet man zweckmäßig in Mengen von 0,005 bis 2, vorzugsweise 0,05 bis 1 Äquivalenten Säure je Mol Ausgangsstoff V.

Die Reaktion der 2. Stufe wird in der Regel bei einer Temperatur von 30 bis 150 °C, vorteilhaft von 70 bis 120 °C, drucklos oder unter Druck, kontinuierlich oder diskontinuierlich durchgeführt. Bevorzugt wird man nach Verfahren a) oder b) und gegebenenfalls mit Methylenchlorid, aromatischen Kohlenwasserstoffen und/oder vorteilhaft 1,1,2-Trichlorethan in der 1. Stufe umsetzen und die Lösungsmittel auch für die 2. Stufe im Gemisch belassen. Man kann auch das Lösungsmittel vor oder nach der Säurezugabe entfernen und dann ein zusätzliches Lösungsmittel zugeben. Man kann ebenfalls das zusätzliche Lösungsmittel ohne Abtrennung des Lösungsmittels der 1. Stufe dem Reaktionsgemisch der 1. Stufe zusetzen. Als zusätzliche Lösungsmittel kommen gegebenenfalls unter den Reaktionsbedingungen inerte organische Lösungsmittel wie aromatische Kohlenwasserstoffe, z. B. Toluol, Xylol, Chlorbenzol, Fluorbenzol, Brombenzol, o-, p- und m-Dichlorbenzol oder entsprechende Gemische in Frage, vorteilhaft in einer Menge von 100 bis 10 000 Gew.%, bezogen auf die Ausgangsstoffe III und IV.

Die Reaktion kann wie folgt durchgeführt werden : Ein Gemisch von Ausgangsstoff V, Lösungsmittel und gegebenenfalls Base wird bei der Reaktionstemperatur gehalten. Dann wird Säure zugegeben, gegebenenfalls das Lösungsmittel der 1. Stufe durch ein anderes für die 2. Stufe ersetzt, und die Reaktion der 2. Stufe in der meist zweiphasigen Mischung durchgeführt, wobei sich die Ausgangsstoffe III, IV und II überwiegend in der organischen Phase befinden und die wäßrige Phase den sauren Katalysator enthält.

Der Ausgangsstoff II wird dann in üblicher Weise, z. B. durch Abtrennen der organischen Phase, Extraktion der wäßrigen Phase und fraktionierte Destillation der vereinigten organischen Phasen, isoliert und mit ihm in der 3. Stufe in vorgenannter Weise die erfindungsgemäße Hydrierung durchgeführt. Die den sauren Katalysator enthaltende wäßrige Phase kann nach erfolgter Umsetzung abgetrennt und beim nächsten Ansatz wieder verwendet werden.

In einer weiteren Ausführungsform kann man auch auf anderem Wege hergestellten Ausgangsstoff III und/oder IV in vorgenannter Weise der 2. Stufe (Säurebehandlung) unterwerfen und den so gebildeten Ausgangsstoff II in vorgenannter Weise hydrieren.

Die nach dem Verfahren der Erfindung herstellbaren Verbindungen sind wertvolle Ausgangsstoffe für die Herstellung von Farbstoffen, Pharmaceutika und Schädlingsbekämpfungsmitteln, z. B. die in EP-Arm. 0 071 707 beschriebenen Herbizide. Bezüglich der Verwendung verweisen wir auf die vorgenannten Veröffentlichungen.

Beispiel 1

a) Herstellung von 3-Formyl-5,6-dihydro-2H-thiopyran

Eine Mischung aus 2 000 ml 1,1,2-Trichlorethan und 1,3 ml Pyridin wurde bei 21 °C mit Schwefelwasserstoff gesättigt. Danach wurden 507 g Acrolein unter gleichzeitigem Einleiten von Schwefelwasserstoff zugetropft, wobei die Temperatur durch Kühlung auf 35 °C gehalten wurde. Anschließend wurde Schwefelwasserstoff in die entstandene Mischung bis zur Sättigung eingeleitet, wobei insgesamt 365 g Schwefelwasserstoff aufgenommen wurden. Nun wurden weitere 507 g Acrolein — ohne Zufuhr von Schwefelwasserstoff — bei 32 °C zugetropft und die erhaltene Lösung 12 Stunden bei 22 °C nachgerührt. Danach wurden 1 000 ml 37 gew.%iger Phosphorsäure zum Reaktionsgemisch gegeben. Das Gemisch wurde 7 Stunden bei 85 °C gerührt, abgekühlt, die organische Phase abgetrennt und die wäßrige Phase zweimal mit jeweils 500 ml 1,1,2-Trichlorethan extrahiert ; die vereinigten organischen Phase wurden über Natriumsulfat getrocknet. Das Lösungsmittel wurde im Vakuum entfernt. Mit dem verbleibenden Rückstand wurde durch eine Vakuumdestillation in einem Dünnfilmverdampfer eine Trennung von hochsiedenen Nebenprodukten erreicht, wobei die erhaltenen 1 060 g 3-Formyl-5,6-dihydro-2H-thiopyran (Kp = 75 bis 78 °C/0,25 mbar ; Fp = 31 °C) direkt in die Hydrierung eingesetzt wurden.

b) Herstellung von 3-Formyl-tetrahydrothiopyran

In einen Rollautoklaven mit einem Inhalt von 3 000 ml wurden 1 792 g 3-Formyl-dihydro-2H-thiopyran, die nach Beispiel 1a) hergestellt wurden, 200 ml Ethylacetat und 220 g Raney-Nickel eingefüllt. Der Autoklav wurde verschlossen und mit Stickstoff gespült. Anschließend wurde Wasserstoff bis zu einem Gesamtdruck von 20 bar aufgepreßt, das Gemisch auf 90 °C erhitzt und unter diesen Bedingungen unter weiterem Einleiten von Wasserstoff bis zur Druckkonstanz hydriert. Danach wurde abgekühlt, der Katalysator abfiltriert und das Filtrat durch Destillation im Vakuum gereinigt. Es wurden 1 658 g 3-Formyl-tetrahydrothiopyran (Kp = 94 bis 95 °C/10 mbar) erhalten (91 % d. Th.). Der Katalysator wurde in siebzehn Umsetzungen 1b) hintereinander ohne Aktivitätsverlust und mit derselben Ausbeute an reinem Endstoff eingesetzt.

Beispiel 2

a) Die Umsetzung wurde analog Beispiel 1a), aber mit Methylenchlorid als Lösungsmittel, durchgeführt, das nach Zugabe der wäßrigen Phosphorsäure abdestilliert und durch dieselbe Menge Toluol ersetzt wurde. Die Umsetzung wurde dann analog Beispiel 1a) in saurem Medium weitergeführt. Man erhielt 974 g 3-Formyl-5,6-dihydro-2H-thiopyran (Kp = 75 bis 78 °C/0,25 mbar ; Fp = 31 °C).

b) Mit dem so erhaltenen Ausgangsstoff II wurde die Hydrierung analog Beispiel 1b) durchgeführt. Man erhielt dieselben Ergebnisse und dieselbe Aktivität des Katalysators wie in Beispiel 1b).

Beispiel 3 (Vergleich)

a) Die Umsetzung wurde analog Beispiel 1a), aber in 1,1,1-Trichlorethan als Lösungsmittel durchgeführt. Beim Zutropfen des Acroleins erfolgt unter stark exothermer Reaktion — im Gegensatz zu Beispiel 1a) — die Bildung von weißen, schmierigen, polymeren Produkten, die sich nicht mehr lösten. Beim Aufarbeiten des Reaktionsansatzes wurden nur gummiartige, hochmolekulare Produkte isoliert.

b) Eine Umsetzung analog Beispiel 1b) war daher nicht durchführbar.

Beispiel 4

In einen Rührautoklaven mit einem Inhalt von 300 ml wurde folgende Mischung eingefüllt : 32 g 3-Formyl-5,6-dihydro-2H-thiopyran, 100 ml Ethylacetat und 3,2 g Katalysator (Zusammensetzung : 7,9 Gew.% Nickel, 7,9 Gew.% Kobalt und 3,2 Gew.% Kupfer auf Aluminiumoxid). Anschließend wurde das Gemisch bei 140 °C und 20 bar Wasserstoffdruck bis zur Druckkonstanz hydriert. Durch Vakuumdestillation wurden 28 g 3-Formyl-tetrahydrothiopyran (86 % d. Th.) isoliert. Der Katalysator wurde ohne Verschlechterung der Ergebnisse in weiteren 17 Umsetzungen wiederverwendet.

Beispiel 5

Die Umsetzung wurde analog Beispiel 4, aber mit 3,2 g Raney-Cobalt als Katalysator und bei einer Hydriertemperatur von 120 °C durchgeführt. Unter diesen Bedingungen wurde 3-Formyl-tetrahydrothiopyran in einer Ausbeute von 88 % der Theorie erhalten. Der Katalysator wurde ohne Verschlechterung der Ergebnisse in weiteren 17 Umsetzungen wiederverwendet.

Beispiel 6 (Vergleich)

Die Umsetzung wurde analog Beispiel 4, mit 3,2 g Katalysator, der 10 Gew.% Palladium auf Aktivkohle enthält, gearbeitet. Es wurden 25 g Reaktionsprodukt erhalten, das nach GC-Analyse folgende Zusammensetzung hatte :

7 % 3-Methyl-tetrahydrothiopyran
86 % 3-Formyl-tetrahydrothiopyran
7 % 3-Formyl-5,6-dihydro-2H-thiopyran. Der Katalysator wurde ein zweites Mal bei der Reaktion eingesetzt, wobei ein weitgehender Aktivitätsverlust festzustellen war ; die Ausbeute betrug nur 12 % der Theorie.

Beispiel 7

In einen Rührautoklaven mit einem Inhalt von 300 ml wurde folgende Mischung eingefüllt : 38,4 g 3-Formyl-5,6-dihydro-2H-thiopyran, 100 g Methanol und 6,5 g Katalysator (Zusammensetzung : 5 Gew.% Platin, 2,5 Gew.% Praseodymoxid auf Aluminiumoxid). Anschließend wurde das Gemisch bei 130 °C und 50 bar Wasserstoffdruck bis zur Druckkonstanz hydriert. Es wurden 37 g Reaktionsprodukt erhalten, das nach GC-Analyse folgende Zusammensetzung hatte :

3 % 3-Methyl-tetrahydrothiopyran
92 % 3-Formyl-tetrahydrothiopyran (87 % der Theorie)
5 % 3-Formyl-5,6-dihydro-2H-thiopyran.

Der Katalysator wurde ohne Verschlechterung der Ergebnisse in weiteren 17 Umsetzungen wiederverwendet.

Beispiel 8

Es wurde analog Beispiel 7 gearbeitet, aber mit 6,5 g Katalysator der Zusammensetzung : 5 Gew.% Platin, 1 Gew.% Silber auf Aluminiumoxid. Als Lösungsmittel wurde Tetrahydrofuran verwendet. Das unter diesen Bedingungen erhaltene Reaktionsprodukt hatte nach gaschromatographischer Analyse folgende Zusammensetzung :

88 % 3-Formyl-tetrahydrothiopyran (83,6 % der Theorie)
3 % 3-Formyl-5,6-dihydro-2H-thiopyran
9 % 3-Hydroxymethyl-tetrahydrothiopyran.

Der Katalysator wurde ohne Verschlechterung der Ergebnisse in weiteren 17 Umsetzungen wiederverwendet.

Beispiel 9

Es wurde analog Beispiel 7 gearbeitet, aber mit 6,5 g Katalysator der Zusammensetzung : 5 % Gew.% Platin auf einem Träger aus 19,4 Gew.% Magnesiumoxid und 80,6 Gew.-% Aluminiumoxid. Unter diesen Bedingungen wurden nach gaschromatographischer Analyse folgende Ergebnisse erhalten :

5 % 3-Methyl-tetrahydrothiopyran
91 % 3-Formyl-tetrahydrothiopyran (86,5 % der Theorie)
1 % 3-Formyl-5,6-dihydro-2H-thiopyran
3 % 3-Hydroxymethyl-tetrahydrothiopyran

Der Katalysator wurde ohne Verschlechterung der Ergebnisse in weiteren 17 Umsetzungen wiederverwendet.

Beispiel 10

a) In eine Lösung von 420 g Acrolein und 42 g Dimethylformamid wurden bei — 5 °C innerhalb von 3

Stunden 130 g Schwefelwasserstoff eingeführt, wobei eine sofortige Umsetzung erfolgte. Das erhaltene Reaktionsgemisch versetzte man bei 20 °C mit 600 ml Toluol und 100 ml 75 gew.%iger Phosphorsäure und erwärmte das Gemisch während 2 Stunden auf 115 °C unter gleichzeitiger Wasserauskreisung. Nach Abtrennung von 65 ml Wasser wurde das Gemisch destilliert. Man erhielt 430 g 3-Formyl-5,6-dihydro-2H-thiopyran (89 % der Theorie).

b) Mit dem so erhaltenen Ausgangsstoff II wurde die Umsetzung analog Beispiel 1b) durchgeführt. Es wurden 1 658 Gewichtsteile 3-Formyltetrahydrothiopyran (Kp = 94 bis 95 °C/10) erhalten (91 % der Theorie). Der Katalysator wurde in 17 Umsetzungen hintereinander ohne Aktivitätsverlust und mit denselben Ausbeuten an reinem Endstoff eingesetzt.

Beispiel 11

a) Herstellung von 3-Formyl-5,6-dihydro-2,6-dimethyl-2H-thiopyran

In eine Mischung aus 500 ml Toluol, 70 g Crotonaldehyd und 10 ml Triethylamin wurden bei 30 °C 17 g Schwefelwasserstoff eingeleitet. Anschließend wurde bei Raumtemperatur 12 Stunden gerührt und dann wurden 300 ml 37 gew.%iger Phosphorsäure zugegeben. Man rührte bei 90 °C 8 Stunden, kühlte auf Raumtemperatur ab und trennte die organische Phase ab. Die wäßrige Phase wurde einmal mit Toluol extrahiert. Die vereinigten organischen Phasen wurden einmal mit Wasser gewaschen, über Natriumsulfat getrocknet und das Lösungsmittel im Vakuum entfernt. Der Rückstand wurde bei 0,6 mbar destilliert. Man erhielt 64 g (82 % der Theorie) 3-Formyl-5,6-dihydro-2,6-dimethyl-2H-thiopyran (Kp = 73 bis 76 °C).

b) Herstellung von 3-Formyl-2,6-dimethyl-tetrahydrothiopyran

In einem Rührautoklaven mit einem Inhalt von 300 ml wurden 100 g 3-Formyl-5,6-dihydro-2,6-dimethyl-2H-thiopyran, die nach Beispiel 11a) hergestellt wurden, 60 ml Essigsäureethylester und 30 g Raney-Nickel eingefüllt. Der Autoklav wurde verschlossen und mit Stickstoff gespült. Anschließend wurde Wasserstoff bis zu einem Gesamtdruck von 30 bar aufgepreßt, das Gemisch auf 90 °C erhitzt und unter diesen Bedingungen bis zur Druckkonstanz hydriert. Danach wurde abgekühlt, der Katalysator abfiltriert und das Filtrat durch Vakuumdestillation gereinigt. Es wurden 89 g 3-Formyl-2,6-dimethyl-tetrahydrothiopyran (Kp = 127 bis 129 °C/10 mbar) erhalten (88 % der Theorie).

Beispiel 12

a) Die Herstellung von 3-Formyl-5,6-dihydro-2H-thiopyrane wird analog Beispiel 1a durchgeführt.

b) Herstellung von 3-Formyl-tetrahydrothiopyran

In einen Rührautoklaven mit einem Inhalt von 3 000 ml wurden 1 792 g 3-Formyl-dihydro-2H-thiopyran, die nach Beispiel 1a) hergestellt wurden, 200 ml N,N-Dimethylformamid und 75 g Raney-Nickel eingefüllt. Der Autoklav wurde verschlossen und mit Stickstoff gespült. Anschließend wurde Wasserstoff bis zu einem Gesamtdruck von 200 bar aufgepreßt, das Gemisch auf 130 °C erhitzt und unter diesen Bedingungen unter weiterem Einleiten von Wasserstoff bis zur Druckkonstanz hydriert. Danach wurde abgekühlt, der Katalysator abfiltriert und das Filtrat durch Destillation im Vakuum gereinigt. Es wurden 1 639 g 3-Formyl-tetrahydrothiopyran (Kp = 94 bis 95 °C/10 mbar) erhalten (90 % d. Th.).

Beispiel 13

a) Die Herstellung des Ausgangsstoffs II und b) die Hydrierung wurden analog Beispiel 12 durchgeführt, wobei die Hydrierung bei einem Druck von 150 bar erfolgte. Man erhielt 1 676 g 3-Formyl-tetrahydrothiopyran (Kp = 94 bis 95 °C/10 mbar) (92 % der Theorie).

**Patentansprüche**

1. Verfahren zur Herstellung von 3-Formyl-tetrahydrothiopyranen der Formel

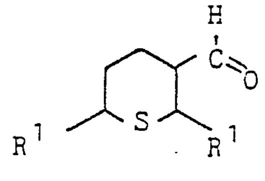

(I)

worin die einzelnen Reste $R^1$ jeweils ein Wasserstoffatom oder einen Alkylrest mit 1 bis 8 Kohlenstoffatomen, der durch unter den Reaktionsbedingungen inerte Gruppen substituiert sein kann, bedeuten, durch Hydrierung von 3-Formyl-5,6-dihydro-2H-thiopyranen in Gegenwart von Katalysatoren, dadurch gekennzeichnet, daß man 3-Formyl-5,6-dihydro-2-H-thiopyrane der Formel

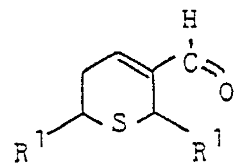

(II)

worin $R^1$ die vorgenannte Bedeutung besitzt, in Gegenwart von Katalysatoren, die Nickel, Kobalt, Platin, Kupfer und/oder Silber enthalten, hydriert.

2. Verfahren nach Anspruch 1, dadurch gekennzeichnet, daß man in einer ersten Stufe Bis-(β-formylethyl)-sulfide der Formel

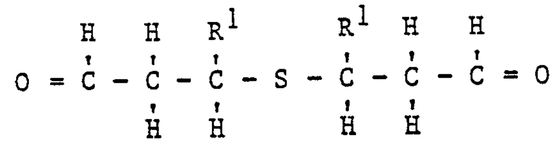

(III)

worin $R^1$ die vorgenannte Bedeutung besitzt, und/oder 3-Formyl-4-hydroxytetrahydrothiopyrane der Formel

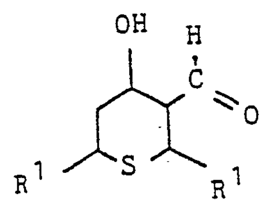

(IV)

worin $R^1$ die vorgenannte Bedeutung besitzt, in Gegenwart saurer Katalysatoren umsetzt und dann in einer zweiten Stufe die so gebildeten 3-Formyl-5,6-dihydro-2H-thiopyrane der Formel

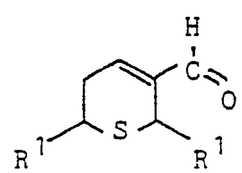

(II)

worin $R^1$ die vorgenannte Bedeutung besitzt, in Gegenwart von Katalysatoren, die Nickel, Kobalt, Platin, Kupfer und/oder Silber enthalten, hydriert.

3. Verfahren nach Anspruch 1, dadurch gekennzeichnet, daß man in einer ersten Stufe Acroleine der Formel

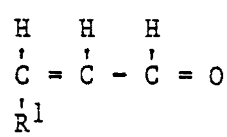

(V)

worin $R^1$ die vorgenannte Bedeutung besitzt, mit Schwefelwasserstoff in Gegenwart von

a) basischen Katalysatoren und von Methylenchlorid, aromatischen Kohlenwasserstoffen und/oder 1,1,2-Trichlorethan als Lösungsmittel und/oder

b) Carbonsäureamiden

umsetzt und dann in einer zweiten Stufe das so erhaltene Gemisch von Bis-(β-formylethyl)-sulfiden der Formel

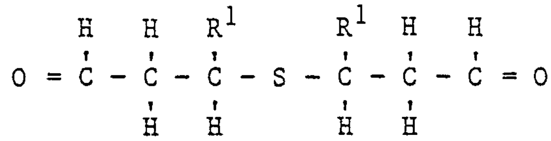

(III)

worin $R^1$ die vorgenannte Bedeutung besitzt, und 3-Formyl-4-hydroxytetrahydrothiopyranen der Formel

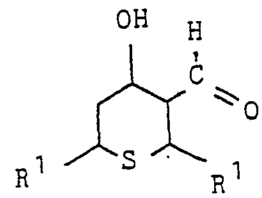

(IV)

worin $R^1$ die vorgenannte Bedeutung besitzt, in Gegenwart saurer Katalysatoren umsetzt und dann in einer dritten Stufe die so gebildeten 3-Formyl-5,6-dihydro-2H-thiopyrane der Formel

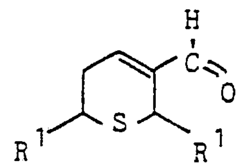

(II)

worin $R^1$ die vorgenannte Bedeutung besitzt, in Gegenwart von Katalysatoren, die Nickel, Kobalt, Platin, Kupfer und/oder Silber enthalten, hydriert.

4. Verfahren nach Anspruch 1, dadurch gekennzeichnet, daß als Hydrierkatalysatoren Nickel-, Kobalt-, Platin-, Kupfer- und/oder Silberkatalysatoren, die noch dazu basische Oxide enthalten, verwendet werden.

**Claims**

1. A process for the preparation of a 3-formyltetrahydrothiopyran of the formula

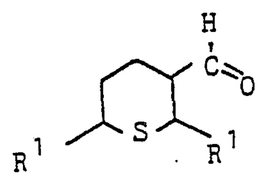

(I)

where the individual radicals $R^1$ are each hydrogen or alkyl of 1 to 8 carbon atoms optionally substituted by groups which are inert under the reaction conditions, by hydrogenation of a 3-formyl-5,6-dihydro-2H-thiopyran in the presence of a catalyst, wherein a 3-formyl-5,6-dihydro-2H-thiopyran of the formula

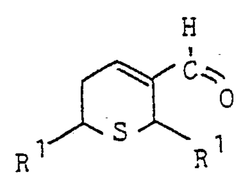

(II)

where $R^1$ has the above meanings, is hydrogenated in the presence of a catalyst containing nickel, cobalt, platinum, copper and/or silver.

2. A process as claimed in claim 1, wherein, in a first stage, a bis-(β-formylethyl) sulfide of the formula

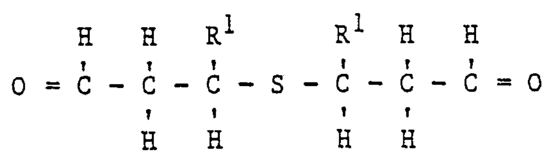

(III)

where $R^1$ has the above meanings, and/or a 3-formyl-4-hydroxytetrahydrothiopyran of the formula

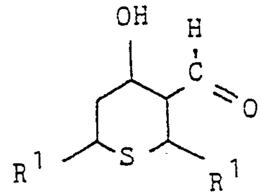

(IV)

where $R^1$ has the above meanings, are reacted in the presence of an acidic catalyst, and then, in a second stage, the resulting 3-formyl-5,6-dihydro-2H-thiopyran of the formula

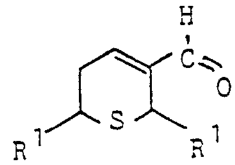

(II)

where $R^1$ has the above meanings, is hydrogenated in the presence of a catalyst containing nickel, cobalt, platinum, copper and/or silver.

3. A process as claimed in claim 1, wherein, in a first stage, an acrolein of the formula

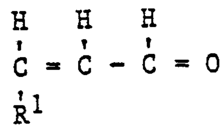

(V)

where $R^1$ has the above meanings, is reacted with hydrogen sulfide

a) in the presence of a basic catalyst and of methylene chloride, an aromatic hydrocarbon and/or 1,1,2-trichloroethane as solvent, and/or

b) in the presence of a carboxamide,

and then, in a second stage, the resulting mixture of a bis-(β-formylethyl) sulfide of the formula

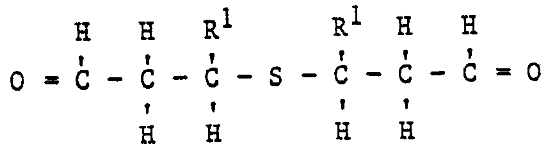

(III)

where $R^1$ has the above meanings, and a 3-formyl-4-hydroxy-tetrahydrothiopyran of the formula

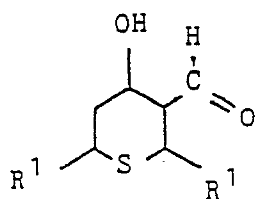

(IV)

where $R^1$ has the above meanings, is reacted in the presence of an acidic catalyst, and then, in a third stage, the resulting 3-formyl-5,6-dihydro-2H-thiopyran of the formula

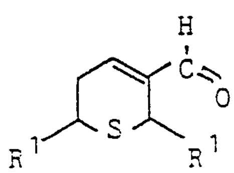

(II)

where $R^1$ has the above meanings, is hydrogenated in the presence of a catalyst containing nickel, cobalt, platinum, copper and/or silver.

4. A process as claimed in claim 1, wherein the hydrogenation catalyst used is a catalyst which contains basic oxides in addition to nickel, cobalt, platinum, copper and/or silver.

**Revendications**

1. Procédé pour la préparation de 3-formyl-tétrahydrothiopyranes répondant à la formule

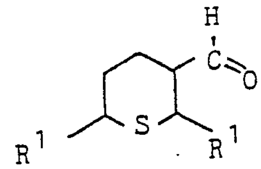

(I)

dans laquelle les groupes individuels $R^1$ représentent chacun un atome d'hydrogène ou un groupe alkyle avec 1 à 8 atomes de carbone qui, dans les conditions de la réaction, peuvent être substitués par des groupes inertes, par hydrogénation de 3-formyl-5,6-dihydro-2H-thiopyrane en présence de catalyseurs, caractérisé en ce que l'on hydrogène un 3-formyl-5,6-dihydro-2H-thiopyrane répondant à la formule

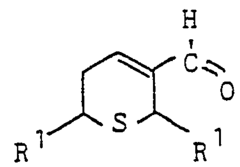

(II)

dans laquelle $R^1$ a la même signification que ci-dessus, en présence de catalyseurs comprenant du nickel, du cobalt, du platine, du cuivre et/ou de l'argent.

2. Procédé selon la revendication 1, caractérisé en ce que, dans une première étape, on fait réagir un bis-(β-formyléthyl)-sulfure répondant à la formule

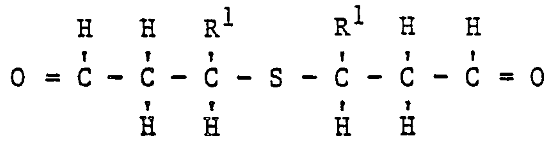

(III)

dans laquelle $R^1$ répond à la définition mentionnée ci-dessus, et/ou un 3-formyl-4-hydroxytétrahydrothiopyrane répondant à la formule

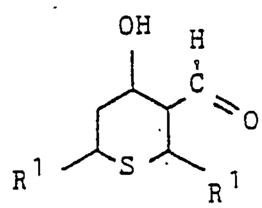

(IV)

dans laquelle $R^1$ répond à la définition mentionnée ci-dessus, en présence de catalyseurs acides, et ensuite dans une deuxième étape, on hydrogène le 3-formyl-5,6-dihydro-2H-thiopyrane ainsi formé, répondant à la formule

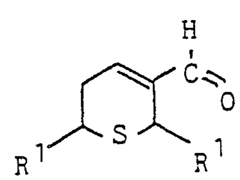

(II)

dans laquelle $R^1$ répond à la définition mentionnée ci-dessus, en présence de catalyseurs comprenant du nickel, du cobalt, du platine, du cuivre et/ou de l'argent.

3. Procédé selon la revendication 1, caractérisé en ce que dans une première étape, on fait réagir de l'acroléine répondant à la formule

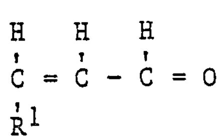

(V)

dans laquelle $R^1$ répond à la définition mentionnée ci-dessus, avec de l'hydrogène sulfuré, en présence

a) de catalyseurs basiques et de chlorure de méthylène, d'hydrocarbures aromatiques et/ou de 1,1,2-trichloréthane comme solvant et/ou

b) d'amides d'acide carboxylique

et ensuite, dans une deuxième étape, on fait réagir le mélange ainsi obtenu, constitué de bis-(β-formyléthyl)-sulfure répondant à la formule

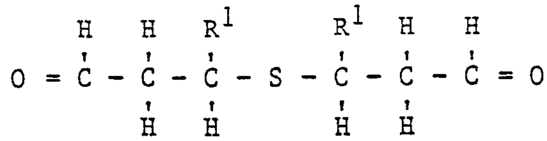

(III)

dans laquelle $R^1$ répond à la définition mentionnée ci-dessus, et de 3-formyl-4-hydroxy-tétrahydrothiopyrane répondant à la formule

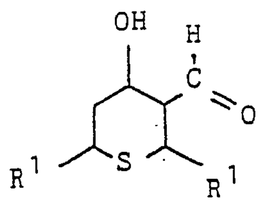

(IV)

dans laquelle $R^1$ répond à la définition mentionnée ci-dessus, en présence de catalyseurs acides, et ensuite, dans une troisième étape, on hydrogène le 3-formyl-5,6-dihydro-2H-thiopyrane répondant à la formule

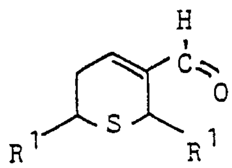

(II)

dans laquelle $R^1$ répond à la définition mentionnée ci-dessus, en présence de catalyseurs contenant du nickel, du cobalt, du platine, du cuivre et/ou de l'argent.

4. Procédé selon la revendication 1, caractérisé en ce que, comme catalyseurs d'hydrogénation, on utilise des catalyseurs contenant du nickel, du cobalt, du platine, du cuivre et/ou de l'argent, et contenant en outre des oxydes basiques.